# EUROPÄISCHE PATENTSCHRIFT

(11) **EP 2 986 563 B1**
(45) Veröffentlichungstag und Bekanntmachung des Hinweises auf die Patenterteilung: **01.03.2017**
(21) Anmeldenummer: 14717770.3
(22) Anmeldetag: 15.04.2014
(51) Int. Cl.: C02F 1/02, C02F 1/20, C02F 1/66, C02F 9/00, C07C 201/16

(54) **VERFAHREN ZUR AUFARBEITUNG VON ABWASSER AUS DER NITROBENZOLHERSTELLUNG**
METHOD FOR THE TREATMENT OF WASTEWATER FROM THE PRODUCTION OF NITROBENZENE
PROCÉDÉ DE TRAITEMENT D'EAUX USÉES ISSUES DE LA FABRICATION DE NITROBENZÈNE

(30) Priorität: 18.04.2013 EP 13164306
(43) Veröffentlichungstag der Anmeldung: 24.02.2016
(73) Patentinhaber: Covestro Deutschland AG, 51373 Leverkusen (DE)
(72) Erfinder: KNAUF, Thomas, 41542 Dormagen (DE); MAIRATA, Antoni, 40549 Düsseldorf (DE); SLUYTS, Erik, B-2930 Brasschaat (BE); VAN TRICHT, Jan, B-2180 Ekeren (BE)
(74) Vertreter: Levpat
(86) Internationale Anmeldenummer: PCT/EP2014/057591
(87) Internationale Veröffentlichungsnummer: WO 2014/170309

(56) Entgegenhaltungen:
- EP-A1- 1 593 654
- WO-A1-2009/027416
- WO-A1-2012/025393
- US-B1- 6 288 289

## Beschreibung

Gegenstand der vorliegenden Erfindung ist ein Verfahren zur Aufarbeitung von alkalischem Abwasser, das bei der Wäsche von rohem, durch Nitrierung von Benzol erhaltenem Nitrobenzol entsteht, wobei
(i) das alkalische Abwasser unter Ausschluss von Sauerstoff bei einem gegenüber Atmosphärendruck erhöhten Druck auf eine Temperatur von 150 °C bis 500 °C erhitzt wird;
(ii) das in (i) erhaltene Abwasser mit einer Base versetzt wird;
(iii) das in (ii) erhaltene Abwasser durch Strippung mit einem Stripp-Gas weiter gereinigt und anschließend der mit Verunreinigungen beladene Stripp-Gasstrom auf eine Temperatur von 10 °C bis 60 °C abgekühlt wird.

Nitrobenzol ist ein wichtiges Zwischenprodukt der chemischen Industrie, das insbesondere zur Herstellung von Anilin und damit auch zur Herstellung der Di- und Polyisocyanate der Diphenylmethanreihe und den darauf basierenden Polyurethanen benötigt wird.

Die Nitrierung von Benzol mit Salpetersäure zu einem Roh-Nitrobenzol war bereits Gegenstand zahlreicher Veröffentlichungen und Patentanmeldungen. Die heute gängigen Verfahren entsprechen im Wesentlichen dem Konzept der adiabaten Nitrierung von Benzol durch ein Gemisch von Schwefel- und Salpetersäure (sog. *Mischsäure*). Ein solches Verfahren wurde erstmals in US 2,256,999 beansprucht und ist in heutigen Ausführungsformen beispielsweise in EP 0 436 443 B1**,** EP 0 771 783 B1 und US 6 562 247 B2 beschrieben. Die Verfahren mit adiabater Reaktionsführung zeichnen sich insbesondere dadurch aus, dass keine technischen Maßnahmen ergriffen werden, um der Reaktionsmischung Wärme zu- oder abzuführen.

Auch sind isotherme Verfahren zur Nitrierung von Benzol mit Mischsäure beschrieben, wie beispielsweise in EP 0 156 199 B1 beschrieben.

Auch sind Verfahren zur Nitrierung von Benzol bekannt, die ohne den Einsatz von Schwefelsäure auskommen. Diese sind beispielsweise beschrieben in US 2 739 174 oder US 3 780 116**.**

Grundsätzlich sind auch Gasphasenverfahren zur Nitrierung von Benzol mit Salpetersäure oder Stickstoffoxiden möglich, jedoch sind die dadurch erzielbaren Ausbeuten noch gering (EP 0 078 247 B1, EP 0 552 130 B1).

All diesen Verfahren ist gemeinsam, dass als Reaktionsprodukt zunächst ein Roh-Nitrobenzol entsteht, das als Verunreinigungen Salpetersäure und - sofern mit Mischsäure nitriert wurde - Schwefelsäure enthält, sowie als organische Verunreinigungen Dinitrobenzol sowie nitrierte Oxidationsprodukte des Benzols, insbesondere nitrierte Phenole (Nitrophenole). Auch enthält es organische Verbindungen, die aus den Verbindungen entstanden, die als Verunreinigungen im eingesetzten Benzol enthalten waren (WO 2008/148608 A1). Darüber hinaus enthält das Roh-Nitrobenzol auch Metallsalze, die in den Säureresten oder im Roh-Nitrobenzol gelöst vorliegen können (DE 10 2007 059 513 A1**).**

Zahlreiche Untersuchungen zielten in der Vergangenheit darauf ab, die Qualität des Roh-Nitrobenzols zu verbessern und somit die Ausbeute bezüglich Benzol und Salpetersäure zu erhöhen. Dank dieser Entwicklungen sind die heutigen adiabaten Flüssigphasenverfahren so ausgereift, dass es allen gelingt, ein Roh-Nitrobenzol herzustellen, das einen geringen Gehalt an Nebenprodukten aufweist, also im Allgemeinen nur zwischen 100 ppm und 300 ppm an Dinitrobenzol und zwischen 1.500 ppm und 2.500 ppm an Nitrophenolen enthält, wobei Pikrinsäure einen Anteil von 10 % bis 50 % an den Nitrophenolen annehmen kann.

Das Roh-Nitrobenzol weist als Verunreinigungen noch Wasser, Benzol sowie Nitrophenole und Dinitrobenzol und - sofern mit Mischsäure nitriert wurde - Schwefelsäure auf. Diese Verunreinigungen sind unerwünscht, da sie in Nachfolgeprozessen, wo Nitrobenzol zum Einsatz kommt, wie zum Beispiel der Herstellung von Anilin, diese negativ beeinflussen können. Geeignete Aufarbeitungsverfahren, die Wasch- und Destillationsstufen beinhalten, sind, z. B. in US 6,288,289 B1**,** EP 1 593 654 A1**,** EP 1 816117 B1 und WO2011/021057 A1 beschrieben.

In EP 1 816 117B1 wird die Aufarbeitung des Roh-Nitrobenzols in einer sauren Wäsche, einer alkalischen Wäsche mit wässriger Natronlauge, einer neutralen Wäsche und einer abschließenden destillativen Reinigung beschrieben. Natürlich können grundsätzlich auch andere Basen als Natronlauge, wie zum Beispiel wässrige Natriumcarbonatlösung oder wässrige Ammoniaklösung (WO 2011/082 977 A1) oder Kaliumhydroxid oder Ammoniak (DE 60 113 579 T2) verwendet werden.

Die Aufarbeitung des alkalischen Abwassers aus der alkalischen Wäsche kann z. B. durch die so genannte "thermische Druckzersetzung" (nachfolgend auch TDZ) erfolgen. Das grundsätzliche Verfahren der TDZ zur Behandlung von aromatische Nitroverbindungen enthaltenden Abwässern ist in folgenden Patenten beschrieben:
In EP 0 005 203 B1 ist ein Verfahren beschrieben zur Aufarbeitung von Nitrohydroxyaromaten enthaltenden Abwässern, wobei die Abwässer unter Luft- und Sauerstoffausschluss bei einem Druck von 50 bar bis 250 bar und mit einer Temperatur von 150 °C bis 500 °C behandelt werden.

In EP 0 503 387 B1 wurde ein ähnliches Verfahren beschrieben, das aber dadurch gekennzeichnet ist, dass das besagte alkalische Abwasser durch Salpetersäurezugabe und anschließende Behandlung in Temperaturbereichen von 180 °C bis 350 °C und einem Druckbereich von 40 bar bis 250 bar aufgearbeitet wird. Beide Verfahren weisen jedoch erhebliche Nachteile auf:
EP 0 005 203 B1 beschreibt nicht die Entfernung von organischen Kohlenwasserstoffen wie Benzol oder Nitrobenzol, die in einem dem technischen Stand entsprechenden adiabaten Nitrierprozess anfallen. Die Reinigung des Abwassers gemäß der Lehre von EP 0 005 203 B1 ist daher entweder unzureichend, oder der Verbrauch an Natronlauge in der TDZ wird sehr hoch.

In EP 0 503 387 B1 gelingt nicht die vollständige Zersetzung von Nitrobenzol, so dass eine weitere Behandlung des Abwassers notwendig ist. Außerdem wird das im Abwasser enthaltene Nitrobenzol in der TDZ zersetzt und mindert daher die erzielte Ausbeute. Die nach der Lehre von EP 0 503 387 B1 erforderliche Anwesenheit von Salpetersäure in der TDZ treibt zudem die Prozesskosten in zweierlei Hinsicht in die Höhe: zum einen durch den Verbrauch an Salpetersäure und zum anderen durch die hohen Materialbelastungen infolge der Korrosivität der Salpetersäure und die damit verbunden hohen Investitionskosten, etwa für einen korrosionsbeständigen mit Titan ausgekleideten Rohrreaktor. Ein zusätzlicher, nicht beschriebener Nachteil ist auch die Notwendigkeit, dass das alkalische Abwasser zunächst einmal neutralisiert werden muss (z. B. mit zusätzlicher Salpetersäure), ehe dieses durch Zugabe der Salpetersäure in einen sauren pH-Bereich überführt werden kann. Es ist also eine verhältnismäßig große Menge an Säure erforderlich.

EP 1 593 654 A1 beschreibt ein Verfahren zur Aufarbeitung von alkalischen Abwässern, die bei der Wäsche von rohem Nitrobenzol entstehen, wobei das rohe Nitrobenzol durch adiabate Nitrierung von Benzol mit Nitriersäure hergestellt und anschließend in einer sauren Wäsche und danach in einer alkalischen Wäsche gewaschen wird, wobei ein alkalisches Abwasser enthaltend Benzol in Konzentrationen von 100 ppm bis 3.000 ppm und Nitrobenzol in Konzentrationen von 1.000 ppm bis 10.000 ppm erhalten wird, wobei aus dem alkalischen Abwasser anschließend nicht gelöst vorliegendes Benzol und/oder Nitrobenzol abgeschieden wird, und optional aus dem alkalischen Abwasser anschließend restliches Benzol und/oder Nitrobenzol durch Strippen entfernt wird, und das alkalische Abwasser anschließend unter Ausschluss von Sauerstoff auf Temperaturen von 150 °C bis 500 °C unter Überdruck erhitzt wird. Das solchermaßen behandelte Abwasser kann daher ohne Verdünnung direkt einer biologischen Kläranlage zugeführt werden. Durch die Zersetzung von Nitroverbindungen in wässrigen Abwässern nach dem Stand der Technik entsteht jedoch Ammoniak, der sich in biologischen Kläranlagen nachteilig auswirkt (siehe auch den folgenden Absatz zu WO2012/025393 A1).

In WO2012/025393 A1 wird die Aufarbeitung von Abwässern, die bei der Aufreinigung von rohen aromatischen Nitroverbindungen nach der Nitrierung von aromatischen Verbindungen erhalten werden, beschrieben, und insbesondere die Problematik der Entfernung von Ammoniak, der bei der thermischen Zersetzung von Nitroverbindungen entsteht, behandelt. Das beschriebene Verfahren sieht folgende Schritte vor: (a) Einstufiges oder mehrstufiges Waschen der rohen aromatischen Nitroverbindung unter Erhalt mindestens einer organischen Phase und mindestens einer wässrigen Phase sowie Abtrennung der wässrigen Phase oder der wässrigen Phasen, wobei Schritt (a) die Zugabe einer Base umfasst, die sich von Ammoniak unterscheidet, und anschließend (b) optional Entfernung organischer Bestandteile aus mindestens einem Teil der in Schritt (a) erhaltenen wässrigen Phase oder wässrigen Phasen durch Strippung vorzugsweise mit Wasserdampf, anschließend (c) Entfernung organischer Verbindungen aus mindestens einem Teil der aus Schritt (a) beziehungsweise Schritt (b) resultierenden wässrigen Phase oder wässrigen Phasen durch thermischen und/oder oxidativen Abbau, anschließend (d) destillative Abreicherung von Ammoniak aus zumindest einem Teil der aus Schritt (c) resultierenden wässrigen Phase oder wässrigen Phasen, und anschließend (e) optional Zuführung zumindest eines Teiles der aus Schritt (d) resultierenden wässrigen Phase oder wässrigen Phasen zu einer biologischen Abwasseraufbereitung. Insbesondere sollte der Ammoniakgehalt im Abwasser in Schritt (d), welches bei der Nitrierung von aromatischen Verbindungen und anschließender Entfernung organischer Bestandteile anfällt, reduziert werden. In diesem Zusammenhang spricht WO 2012/025393 A1 von freiem Ammoniak und nicht von gelösten Ammoniumionen im Abwasser nach der TDZ, das einer biologischen Abwasseraufbereitung zugeführt wird. Aus dem im alkalischen Abwasser vorhandenen Kohlendioxid und Ammoniak bildet sich nämlich immer auch zu einem gewissen Teil Ammoniumcarbonat, das infolge seines Salzcharakters nicht durch Strippung entfernt werden kann wie Ammoniak.

Es bestand daher ein Bedarf an einem weiterentwickelten Verfahren zur Aufarbeitung von alkalischen Abwässern, die bei der Wäsche von durch adiabate Nitrierung von Benzol hergestelltem rohem Nitrobenzol entstehen. Insbesondere sollte das an sich bewährte Verfahren der thermischen Druckzersetzung (TDZ) angewendet werden können, ohne dass der Gehalt an Ammoniak und Ammoniumionen im dabei entstehenden Abwasser Probleme in der weiteren Aufarbeitung etwa in einer biologischen Kläranlage bereitet.

Diesem Bedarf Rechnung tragend ist ein Gegenstand der vorliegenden Erfindung ein Verfahren zur Aufarbeitung von alkalischem Abwasser, das bei der Wäsche von rohem, durch - bevorzugt adiabate - Nitrierung von Benzol erhaltenem Nitrobenzol entsteht, wobei
(i) das alkalische Abwasser unter Ausschluss von Sauerstoff bei einem gegenüber Atmosphärendruck erhöhten Druck, bevorzugt bei einem absoluten Druck von 50 bar bis 350 bar, besonders bevorzugt von 50 bar bis 200 bar, ganz besonders bevorzugt von 70 bar bis 130 bar, auf eine Temperatur von 150 °C bis 500 °C, bevorzugt von 250 °C bis 350 °C, besonders bevorzugt von 270 °C bis 290 °C, für bevorzugt einen Zeitraum von 5 Minuten bis 120 Minuten, besonders bevorzugt von 15 Minuten bis 30 Minuten, erhitzt und anschließend bevorzugt auf eine Temperatur von 60 °C bis 100 °C abgekühlt wird;
(ii) das in (i) erhaltene Abwasser mit einer Base, bevorzugt einer wässrigen Lösung einer Base ausgewählt aus der Gruppe bestehend aus Lithiumhydroxid, Natriumhydroxid, Kaliumhydroxid und Rubidiumhydroxid, besonders bevorzugt einer wässrigen Natriumhydroxid-Lösung, versetzt wird, und zwar bevorzugt so, dass sich ein pH-Wert von mindestens 12, bevorzugt 12 bis 13, einstellt;
(iii) das in (ii) erhaltene Abwasser durch Strippung mit einem Stripp-Gas, bevorzugt Wasserdampf, bevorzugt bei einem absoluten Druck von 0,1 bar bis 5 bar, besonders bevorzugt 0,5 bar bis 2 bar und bevorzugt bei einer Temperatur von 40 °C bis 160 °C, besonders bevorzugt 80 °C bis 120 °C, weiter gereinigt und anschließend der mit Verunreinigungen beladene Stripp-Gasstrom auf eine Temperatur von 10 °C bis 60 °C, bevorzugt von 20 °C bis 50 °C, besonders bevorzugt von 25 °C bis 45 °C, ganz besonders bevorzugt von 30 °C bis 40 °C, abgekühlt wird.

Unter *Strippung* ist erfindungsgemäß die Entfernung von bestimmten flüchtigen Bestandteilen aus Flüssigkeiten durch das Durchleiten eines Stripp-Gases (Luft, Wasserdampf, etc.) zu verstehen, wobei die genannten Bestandteile in die Gasphase überführt werden (d. h., mit dem durchgeleiteten Gas aus der Flüssigkeit ausgetragen werden).

Nachfolgend werden Ausführungsformen der Erfindung näher beschrieben. Dabei sind verschiedene Ausführungsformen beliebig miteinander kombinierbar, sofern sich aus dem Kontext nicht eindeutig das Gegenteil ergibt.

In einer besonders bevorzugten Ausführungsform umfasst der Gesamtprozess die folgenden Schritte:
a) Nitrierung von Benzol mit Salpetersäure oder - bevorzugt - einer Mischung aus Salpetersäure und Schwefelsäure (nachfolgend auch Mischsäure genannt) und Abtrennung der wässrigen Phase;
b) Wäsche des in Schritt a) erhaltenen organischen Verfahrensprodukts;
c) alkalische Wäsche des in Schritt b) erhaltenen gewaschenen organischen Verfahrensprodukts, wobei bevorzugt ein alkalisches Abwasser enthaltend Benzol in einer Konzentration von 100 ppm bis 3.000 ppm und Nitrobenzol in einer Konzentration von 1.000 ppm bis 10.000 ppm erhalten wird;
d) optionale Abscheidung von Benzol und/oder Nitrobenzol aus dem in Schritt c) erhaltenen alkalischem Abwasser;
e) optionale Strippung des in Schritt c) oder Schritt d) erhaltenen alkalischen Abwassers zur Entfernung von Restmengen an Benzol und/oder Nitrobenzol;
f) Aufarbeitung des in Schritt c) oder Schritt d) oder Schritt e) erhaltenen alkalischen Abwassers umfassend die oben bezeichneten Schritte (i) bis (iii).

Die Nitrierung von Benzol zu Nitrobenzol mit Salpetersäure oder einem Gemisch aus Salpetersäure und Schwefelsäure (Mischsäure) in **Schritt a)** erfolgt dabei nach einem beliebigen der dem Fachmann bekannten Verfahren aus dem Stand der Technik, wie z. B. in EP 0 436 443 B1, EP 0 771 783 B1, US 6 562 247 B2 oder in EP 0 156 199 B1 beschrieben. Da in allen Verfahren des Standes der Technik ein Roh-Nitrobenzol erhalten wird, das überschüssige Säure, nicht umgesetztes Benzol, Wasser und organische Nebenkomponenten enthält, ist die erfindungsgemäße Reinigung des in Schritt a) erhaltenen Roh-Nitrobenzols grundsätzlich auf alle Verfahren anwendbar. Z. B. kann die Nitrierung unter Abfuhr der Reaktionswärme (d. h., isotherm oder angenähert isotherm) oder auch ohne Abfuhr der Reaktionswärme in bevorzugt isolierten Reaktoren (d. h., adiabat) erfolgen. Bevorzugt ist jedoch die Umsetzung von Benzol mit einem Gemisch aus Salpetersäure und Schwefelsäure unter adiabater Verfahrensführung, wie sie insbesondere in DE 10 2008 048 713 A1, und dort insbesondere in Absatz [0024], beschrieben ist. Das in Schritt a) hergestellte rohe Nitrobenzol wird schließlich in einem Trennbehälter von überschüssiger Säure (bei Verwendung von Mischsäure im Wesentlichen Schwefelsäure) getrennt.

Die in Schritt a) nach der Phasentrennung erhaltene organische Phase, die üblicherweise noch Spuren an Säure enthält, wird in **Schritt b)** in einer bis zwei, bevorzugt einer Wäsche(n) gewaschen und dann von der sauren wässrigen Phase durch Phasentrennung getrennt (bei mehreren Wäschen nach jeder einzelnen Wäsche). In Schritt b) werden die Säurereste, die das rohe Nitrobenzol enthält, ausgewaschen; daher wird dieser Verfahrensschritt auch als *saure Wäsche* bezeichnet. Bevorzugt wird dabei so verfahren, dass sich in der nach der Phasentrennung erhaltenen wässrigen Phase ein pH-Wert < 5 (gemessen bei 20 °C) eingestellt. Als Waschflüssigkeit kann in Schritt b) jede Art von Wasser, z. B. vollentsalztes Wasser oder Dampfkondensat, eingesetzt werden. Das Wasser kann auch gelöste Salze enthalten. Bevorzugt werden zur Durchführung von Schritt b) im Betrieb anfallende wässrige Ströme rezyklisiert.

Die so erhaltene organische Phase wird anschließend in **Schritt c)** in bevorzugt einer bis zwei, besonders bevorzugt einer alkalischen Wäsche(n) mit einer Base, bevorzugt einer wässrigen Lösung einer Base ausgewählt aus der Gruppe bestehend aus Natriumhydroxid, Natriumcarbonat und Natriumhydrogencarbonat, gewaschen und anschließend von dem alkalischen Waschwasser durch Phasentrennung getrennt (bei mehreren Wäschen nach jeder einzelnen Wäsche). Bevorzugt wird als wässrige Baselösung Natronlauge eingesetzt. Die alkalische Wäsche wird im Folgenden anhand von Natronlauge beschrieben; für den Fachmann ist es ein Leichtes, bei Einsatz anderer Basen erforderlichenfalls entsprechende Abänderungen vorzunehmen.

Bevorzugt hat die eingesetzte Natronlauge einen pH-Wert zwischen 9,0 und 14 (gemessen bei 20 °C). Das Massenverhältnis von Natronlauge zu organischer Phase (im Wesentlichen Nitrobenzol) ist abhängig vom in Schritt a) eingesetzten Benzolüberschuss und liegt bevorzugt zwischen 1 : 80 und 1 : 500. Der pH-Wert der eingesetzten Natronlauge und ihr Massenverhältnis zur organischen Phase werden so eingestellt, dass acide Verunreinigungen (z. B. als Nebenprodukte gebildete Nitrophenole und in Schritt b) nicht vollständig entfernte Säurereste) in Schritt c) weitgehend bis vollständig, bevorzugt vollständig, neutralisiert werden.

Im optionalen **Schritt d)** wird aus dem alkalischen Abwasser aus Schritt c) noch enthaltenes, nicht gelöst vorliegendes Benzol und/oder Nitrobenzol abgetrennt. Das so abgetrennte Benzol und/oder Nitrobenzol wird dann bevorzugt wieder dem Nitrierprozess, besonders bevorzugt dem rohen Nitrobenzol, zugeführt. Die Abtrennung des nicht gelöst vorliegenden Nitrobenzols kann dabei durch Abscheider, Absetzbehälter oder andere Phasentrennapparaturen erfolgen. Bevorzugt wird ein Absetzbehälter eingesetzt. Bevorzugt wird in Schritt d) ein alkalisches Abwasser erhalten, das Benzol in einer Konzentration von 100 ppm bis 1.000 ppm und Nitrobenzol in einer Konzentration von 1.200 ppm bis 3.000 ppm enthält. Es ist bevorzugt, Schritt d) durchzuführen.

Optional kann anschließend in **Schritt e)** aus dem aus Schritt c) bzw., je nachdem, ob Schritt d) durchgeführt wird oder nicht, aus Schritt d) erhaltenen alkalischen Abwasser Benzol und gegebenenfalls restliches Nitrobenzol durch Strippen entfernt werden. Die Strippung erfolgt dabei vorzugsweise in einer Strippkolonne durch Abstrippen der Restmengen an Benzol und Nitrobenzol mit Wasserdampf über Kopf. Die anfallenden, Benzol und Nitrobenzol enthaltenden Brüden werden dann bevorzugt in die alkalische Wäsche in Schritt c) zurückgeführt. Eine Fehlfunktion der Strippkolonne kann beispielsweise durch redundante Sicherheitseinrichtungen überwacht werden. Bevorzugt wird in Schritt e) ein alkalisches Abwasser erhalten, das Benzol nur noch in einer Konzentration von bis zu 10 ppm und Nitrobenzol in einer Konzentration von bis zu 10 ppm enthält.

In **Schritt f) (i)** (der TDZ) wird das aus den Schritten c, d) bzw. e) erhaltene alkalische Abwasser, das noch mit organischen Salzen der Nitrohydroxyaromaten beladen ist, unter Ausschluss von Sauerstoff bei einem gegenüber Atmosphärendruck erhöhten Druck, bevorzugt bei einem absoluten Druck von 50 bar bis 350 bar, besonders bevorzugt von 50 bar bis 200 bar, ganz besonders bevorzugt von 70 bar bis 130 bar, auf eine Temperatur von 150 °C bis 500 °C, bevorzugt von 250 °C bis 350 °C, besonders bevorzugt von 270 °C bis 290 °C, erhitzt. Es ist auch möglich das alkalische Abwasser unter Inertgasatmosphäre bzw. unter einem Inertgas-Vordruck von beispielsweise 0,1 bar bis 100 bar zu erhitzen. Als Inertgase sind z. B. Stickstoff und/oder Argon geeignet. Je nach Temperatur und gegebenenfalls Inertgas-Vordruck stellen sich beim Erhitzen der Abwässer bevorzugt die zuvor genannten Drücke ein. Die Erhitzung des alkalischen Abwassers und thermische Druckzersetzung der organischen Bestandteile wie Benzol, Nitrobenzol und Nitrohydroxyaromaten erfolgt dabei üblicherweise für 5 Minuten bis 120 Minuten, bevorzugt 15 Minuten bis 30 Minuten. Bevorzugt wird das alkalische Abwasser anschließend so abgekühlt, dass es die TDZ mit einer Temperatur von 60 °C bis 100 °C verlässt.

Die Schritte d), e) und f) (i) können gemäß dem Stand der Technik, bevorzugt gemäß der Offenbarung der EP 1 593 654 A1, durchgeführt werden.

In **Schritt f) (ii)** wird das in f) (i) erhaltene Abwasser mit einer Base, bevorzugt einer wässrigen Lösung einer Base ausgewählt aus der Gruppe bestehend aus Lithiumhydroxid, Natriumhydroxid, Kaliumhydroxid und Rubidiumhydroxid, besonders bevorzugt einer wässrigen Natriumhydroxid-Lösung, versetzt, und zwar bevorzugt so, dass sich ein pH-Wert von mindestens 12, bevorzugt von 12 bis 13, einstellt. Hierdurch wird sichergestellt, dass in dem in Schritt f) (i) erhaltenen Abwasser vorhandene Carbonate im Abwasser verbleiben, und zwar bevorzugt zu > 99,999 %, und kein Kohlendioxid gebildet wird. Das erfindungsgemäße Verfahren ermöglicht es, den Kohlendioxidgehalt im in Schritt f) (ii) erhaltenen Abwasser auf < 10 ppm, bezogen auf die Gesamtmasse des in Schritt f) (ii) erhaltenen Abwassers, zu begrenzen. Durch den hohen pH-Wert in Schritt f) (ii) wird Ammoniak aus Ammoniumcarbonat freigesetzt. Ammoniak kann im Gegensatz zu Ammoniumcarbonat leicht durch Strippung entfernt werden.

In **Schritt f) (iii)** wird schließlich das in f) (ii) erhaltene Abwasser durch Strippung weiter gereinigt und anschließend der mit Verunreinigungen beladene Stripp-Gasstrom auf eine Temperatur von 10 °C bis 60 °C, bevorzugt von 20 °C bis 50 °C, besonders bevorzugt von 25 °C bis 45 °C, ganz besonders bevorzugt von 30 °C bis 40 °C, abgekühlt. Hierbei werden Ammoniak und noch vorhandene organische Bestandteile entfernt. Bevorzugt erfolgt die Strippung im Rahmen der vorliegenden Erfindung im Gegenstrom in einer Strippkolonne, wobei der Gasstrom aus dem eingesetzten Stripp-Gas (bevorzugt Wasserdampf) und den abgestrippten leichtflüchtigen Bestandteilen bevorzugt am Kopf der Strippkolonne austritt (Brüden) und das gestrippte Abwasser bevorzugt am Sumpf der Strippkolonne entnommen wird. Der am Kopf der Strippkolonne entnommene Gasstrom (Brüden) wird in einem Kondensator auf eine Temperatur von 10 °C bis 60 °C, bevorzugt von 20 °C bis 50 °C, besonders bevorzugt von 25 °C bis 45 °C, ganz besonders bevorzugt von 30 °C bis 40 °C abgekühlt und der so gebildete flüssige Strom bevorzugt anschließend in einen Phasentrennapparat überführt, aus dem die sich abscheidenden Organika ausgeschleust werden. Der so erhaltene von Organika weitgehend befreite Strom (in der bevorzugten Ausführungsform mit Wasserdampf als Stripp-Gas, das sog. *Brüdenwasser)* wird bevorzugt teilweise bis vollständig auf den Kopf der Strippkolonne zurückgefahren. Der verbleibende Teil des von Organika weitgehend befreiten Stroms wird bevorzugt direkt (ohne weitere zwischengeschaltete Reinigungsschritte) einer Abwasseraufbereitung, bevorzugt einer biologischen Kläranlage, zugeführt. Die Strippkolonne ist vorzugsweise eine röhrenförmige Einrichtung mit mehreren Einbauten (z. B. Schüttungen aus Füllkörpern, strukturierte Packungen oder Stoffaustauschböden) zum intensiven Stoffaustausch von gasförmiger und flüssiger Phase. Entsprechende Verfahren und Kolonnen sind dem Fachmann bekannt und z. B. in W. Meier, Sulzer, Kolonnen für Rektifikation und Absorption, in: Technische Rundschau Sulzer, 2 (1979), Seite 49 bis 61, beschrieben. Bevorzugt wird die Strippung gemäß **Schritt f) (iii)** bei einem absoluten Druck von 0,1 bar bis 5 bar, besonders bevorzugt 0,5 bar bis 2 bar, und bevorzugt bei einer Temperatur von 40 °C bis 160 °C, besonders bevorzugt 80 °C bis 120 °C, durchgeführt.

Der in Schritt **f) (iii)** gestrippte Ammoniak kann mittels eines Inertgases, bevorzugt Stickstoff, durch Einperlung am Kopf der Strippkolonne in das Abgassystem getrieben und einer technischen Abluftreinigung zugeführt werden.

Der Fachmann würde gemäß dem Stand der Technik die Temperaturen in allen Schritten nach der TDZ stets höher als 60 °C halten, um das Problem von ausfallendem Ammoniumcarbonat zu vermeiden. Dadurch wird allerdings die Möglichkeit, die Organika im Phasentrennapparat am Kopf des Strippers abzutrennen, verschlechtert, weil sich Organika bei erhöhter Temperatur besser in Wasser lösen und sich folglich ein erhöhter Pegel an organischen Bestandteilen im Abwasser, das zur biologischen Kläranlage geleitet wird, einstellt.

Das erfindungsgemäße Verfahren ist durch eine Reihe von Merkmalen charakterisiert, die sich nicht im Stand der Technik finden. So wird bei der erfindungsgemäßen Aufarbeitung des alkalischen Abwassers nach Zugabe von Base nach der TDZ und damit Sicherstellung eines sehr hohen pH-Wertes Kohlendioxid als Carbonat in der wässrigen Phase gehalten. Somit kann im weiteren Aufarbeitungsschritt in der Strippung kein Ammoniumcarbonat durch Reaktion von Ammoniak mit Kohlendioxid entstehen, das zum Beispiel zu Verstopfungen in dem Abgassystem der Strippkolonne führen kann. Des Weiteren kann man nun die Brüden am Kopf der Strippkolonne abkühlen ohne störendes Ammoniumcarbonat zu bilden. Man erzielt eine bessere Phasenseparation im Phasentrennapparat der Strippkolonne zwischen organischer Phase und wässriger Phase, enthaltend Ammoniak.

Erfindungsgemäß gereinigtes Abwasser (d. h., das in Schritt (iii) gestrippte Abwasser, das in der bevorzugten Ausführungsform dem Sumpf der Strippkolonne entnommen wird) lässt sich ohne Probleme direkt, d. h., ohne zwischengeschaltete weitere Reinigungsschritte, einer Abwasseraufbereitung, bevorzugt einer biologischen Kläranlage zuführen.

### Beispiele

### Messmethoden

Gehalt an organischen Komponenten: Gaschromatographie (GC), angegeben sind Flächen-%.
Ammoniumionen-Gehalt: Photometrische Messung nach DIN 38406; angegeben sind Massenanteile in ppm.

### Allgemeine Bedingungen für die Herstellung von Nitrobenzol

Nitrobenzol wurde in einem adiabaten Prozess, wie in EP2168942 A1 beschrieben, hergestellt. Das dabei in der letzten alkalischen Wäsche anfallende Abwasser wurde in den nachfolgenden Beispielen verwendet.

### Beispiel 1 (Vergleich - hohe Kondensationstemperatur, keine Basenzugabe nach TDZ):

Das Abwasser aus der alkalischen Wäsche wurde in einen Absetztank gefahren, in dem ungelöstes Benzol und Nitrobenzol abgeschieden wurden. 3,5 Tonnen pro Stunde an Abwasser, das einen Gehalt an Nitrobenzol von 2.293 ppm, an Benzol von 212 ppm und an Nitrophenolen an 16.244 ppm und einen pH-Wert von 13,4 hatte (2 % NaOH-Überschuss gegenüber dem Gehalt an Nitrophenolen), wurden von dort in die TDZ gefahren und bei einer Verweilzeit von 20 min, einer Temperatur von 290 °C und einem absoluten Druck von 90 bar behandelt. Das entstandene Abwasser wurde nach der TDZ auf 88 °C abgekühlt. Dieses Abwasser hatte einen pH-Wert von 10,3 (0,1 % NaOH bezogen auf Abwasser) und einen sehr hohen Gehalt an freiem Ammoniak (2.300 ppm) und an Carbonat (1,58 %). Anschließend wurde dieses Abwasser mit Direktdampf gestrippt. Im Sumpf der Strippkolonne wurde, bei einem absoluten Druck von 1,02 bar in der Stripperkolonne, ein Strom von 3,9 Tonnen pro Stunde erhalten, der im Wesentlichen Wasser, Ammoniak, Kohlendioxid und Organika enthielt. Das Kopfprodukt der Strippkolonne wurde kondensiert und auf 65 °C abgekühlt. Aus dem Kondensat wurde ein Purge-Strom an Organika ausgeschleust. 0,25 Tonnen pro Stunde des an Organika abgereicherten wässrigen Kondensat-Stromes wurden als Rückfluss in die Strippkolonne zurückgeführt. Der Anteil an Organika, gemessen als TOC (gesamter organischer Kohlenstoff), im erhaltenen Abwasser (Sumpfstrom der Stripp-Kolonne), die einer biologischen Kläranlage zugeführt wurden, betrug 5.945 ppm. Der Gehalt an Ammonium in diesem Abwasser war 182 ppm.

### Beispiel 2 (Vergleich - niedrige Kondensationstemperatur, keine Basenzugabe nach TDZ):

Beispiel 2 wurde durchgeführt wie Beispiel 1 mit dem Unterschied, dass das Kopfprodukt der Strippkolonne kondensiert und auf 35 °C abgekühlt wurde. Der Anteil an Organika im erhaltenen Abwasser, das einer biologischen Kläranlage zugeführt wurde, betrug 5.661 ppm. Es gab massive Probleme mit Verstopfungen im Abgassystem der Strippkolonne, die auf Ablagerungen von Ammoniumcarbonat an Ventilen und in den Rohrleitungen des Abgassystems zurückzuführen waren. Der Gehalt an Ammonium in diesem Abwasser war 212 ppm.

### Beispiel 3 (Vergleich - hohe Kondensationstemperatur, Basenzugabe nach TDZ):

Beispiel 3 wurde bis einschließlich des Prozessschrittes der TDZ durchgeführt wie Beispiel 1. Das auf 88 °C abgekühlte Abwasser aus der TDZ wurde mit 30%iger Natronlauge (100 L/h) versetzt. Der pH-Wert betrug danach 12,5. Danach wurde das Abwasser mit Direktdampf gestrippt. Im Sumpf der Strippkolonne wurde ein Strom von 4,0 Tonnen pro Stunde bei einem absoluten Druck von 1,02 bar erhalten, der im Wesentlichen Wasser, Ammoniak, Kohlendioxid und Organika enthielt. Das Kopfprodukt wurde kondensiert und auf 65 °C abgekühlt. Aus dem Kondensat wurde ein Purge-Strom an Organika ausgeschleust. 0,25 Tonnen pro Stunde des an Organika abgereicherten wässrigen Kondensat-Stromes wurden als Rückfluss in die Strippkolonne zurückgeführt. Der Anteil an Organika im Abwasser, das einer biologischen Kläranlage zugeführt wurde, betrug 5.894 ppm. Der Gehalt an Ammonium war kleiner als 91 ppm.

### Beispiel 4(erfindungsgemäß - niedrige Kondensationstemperatur, Basenzugabe nach TDZ)

Beispiel 4 wurde durchgeführt wie Beispiel 3 mit dem Unterschied, dass das Kopfprodukt der Strippkolonne kondensiert und auf 35 °C abgekühlt wurde. Der Anteil an Organika im erhaltenen Abwasser, das einer biologischen Kläranlage zugeführt wurde, betrug 4.726 ppm. Der Gehalt an Ammonium im Abwasser war kleiner als 87 ppm. Es gab keinerlei Probleme mit Ablagerungen im Bereich des Abgases der Strippkolonne.

Die Beispiele zeigen, dass nur die erfindungsgemäße Vorgehensweise mit Basenzugabe (**Schritt (ii)**) und niedriger Kondensationstemperatur (**Schritt (iii)**) zu geringen Organika-Gehalten von unter 5.000 ppm und geringen Ammonium-Gehalten von unter 90 ppm führt.

## Patentansprüche

1. Verfahren zur Aufarbeitung von alkalischem Abwasser, das bei der Wäsche von rohem, durch Nitrierung von Benzol erhaltenem Nitrobenzol entsteht, wobei
(i) das alkalische Abwasser unter Ausschluss von Sauerstoff bei einem gegenüber Atmosphärendruck erhöhten Druck auf eine Temperatur von 150 °C bis 500 °C erhitzt wird;
(ii) das in (i) erhaltene Abwasser mit einer Base versetzt wird;
(iii) das in (ii) erhaltene Abwasser durch Strippung mit einem Stripp-Gas weiter gereinigt und anschließend der mit Verunreinigungen beladene Stripp-Gasstrom auf eine Temperatur von 10 °C bis 60 °C abgekühlt wird.

2. Verfahren nach Anspruch 1, bei dem die Erhitzung des alkalischen Abwassers in Schritt (i) bei einem absoluten Druck von 50 bar bis 350 bar durchgeführt wird.

3. Verfahren nach Anspruch 1 oder 2, bei dem die Erhitzung des alkalischen Abwassers in Schritt (i) für einen Zeitraum von 5 Minuten bis 120 Minuten durchgeführt wird.

4. Verfahren nach Anspruch 3, bei dem das alkalische Abwasser im Anschluss an die Erhitzung auf eine Temperatur von 60 °C bis 100 °C abgekühlt wird.

5. Verfahren nach einem der Ansprüche 1 bis 4, bei dem die in Schritt (ii) eingesetzt Base eine wässrige Lösung einer Base ausgewählt aus der Gruppe bestehend aus Lithiumhydroxid, Natriumhydroxid, Kaliumhydroxid und Rubidiumhydroxid ist.

6. Verfahren nach einem der Ansprüche 1 bis 5, bei dem die Base in Schritt (ii) so zugegeben wird, dass sich ein pH-Wert von mindestens 12 einstellt.

7. Verfahren nach einem der Ansprüche 1 bis 6, bei dem die Strippung in Schritt (iii) bei einem absoluten Druck von 0,5 bar bis 2 bar und bei einer Temperatur von 80 °C bis 120 °C durchgeführt wird.

8. Verfahren nach einem der Ansprüche 1 bis 7, bei dem das Stripp-Gas Wasserdampf ist.

9. Verfahren nach einem der Ansprüche 1 bis 8, bei dem aus dem in Schritt (iii) nach der Abkühlung des mit Verunreinigungen beladenen Stripp-Gasstroms auf eine Temperatur von 10 °C bis 60 °C erhaltenen Strom organische Bestandteile ausgeschleust werden, so dass ein an organischen Bestandteilen abgereicherter Strom erhalten wird.

10. Verfahren nach Anspruch 9, bei dem der an organischen Bestandteilen abgereicherte Strom teilweise bis vollständig in die Strippung (iii) zurückgeführt wird.

11. Verfahren nach Anspruch 9 oder 10, bei dem der an organischen Bestandteilen abgereicherte Strom, sofern er nicht in die Strippung (iii) zurückgeführt wird, direkt einer Abwasseraufbereitung zugeführt wird.

12. Verfahren nach einem der Ansprüche 1 bis 11, bei dem das in Schritt (iii) erhaltene gestrippte Abwasser direkt einer Abwasseraufbereitung zugeführt wird.

13. Verfahren nach einem der Ansprüche 1 bis 12, bei dem das in Schritt (i) eingesetzte alkalische Abwasser aus Schritt c) oder Schritt d) oder Schritt e) der folgenden Verfahrensschritte stammt:
a) Nitrierung von Benzol mit Salpetersäure oder einer Mischung aus Salpetersäure und Schwefelsäure und Abtrennung der wässrigen Phase;
b) Wäsche des in Schritt a) erhaltenen organischen Verfahrensprodukts;
c) alkalische Wäsche des in Schritt b) erhaltenen gewaschenen organischen Verfahrensprodukts;
d) optionale Abscheidung von Benzol und/oder Nitrobenzol aus dem in Schritt c) erhaltenen alkalischem Abwasser;
e) optionale Strippung des in Schritt c) oder Schritt d) erhaltenen alkalischen Abwassers.

## Claims

1. Process for working up alkaline waste water which is formed during washing of crude nitrobenzene obtained by nitration of benzene, wherein
(i) the alkaline waste water is heated to a temperature of from 150 °C to 500 °C under an increased pressure with respect to atmospheric pressure with exclusion of oxygen;
(ii) a base is added to the waste water obtained in (i);
(iii) the waste water obtained in (ii) is purified further by stripping with a stripping gas and the stripping gas stream loaded with impurities is then cooled to a temperature of from 10 °C to 60 °C.

2. Process according to claim 1, in which the heating of the alkaline waste water in step (i) is carried out under an absolute pressure of from 50 bar to 350 bar.

3. Process according to claim 1 or 2, in which the heating of the alkaline waste water in step (i) is carried out for a period of from 5 minutes to 120 minutes.

4. Process according to claim 3, in which after the heating the alkaline waste water is cooled to a temperature of from 60 °C to 100 °C.

5. Process according to one of claims 1 to 4, in which the base used in step (ii) is an aqueous solution of a base chosen from the group consisting of lithium hydroxide, sodium hydroxide, potassium hydroxide and rubidium hydroxide.

6. Process according to one of claims 1 to 5, in which the base is added in step (ii) such that a pH of at least 12 is established.

7. Process according to one of claims 1 to 6, in which the stripping in step (iii) is carried out under an absolute pressure of from 0.5 bar to 2 bar and at a temperature of from 80 °C to 120 °C.

8. Process according to one of claims 1 to 7, in which the stripping gas is steam.

9. Process according to one of claims 1 to 8, in which organic constituents are purged out of the stream obtained in step (iii) after cooling of the stripping gas stream loaded with impurities to a temperature of 10 °C to 60 °C, such that a stream depleted in organic constituents is obtained.

10. Process according to claim 9, in which the stream depleted in organic constituents is partially to completely returned to the stripping (iii).

11. Process according to claim 9 or 10, in which the stream depleted in organic constituents, where it is not returned to the stripping (iii), is sent directly to a waste water treatment.

12. Process according to one of claims 1 to 11, in which the stripped waste water obtained in step (iii) is sent directly to a waste water treatment.

13. Process according to one of claims 1 to 12, in which the alkaline waste water used in step (i) originates from step c) or step d) or step e) of the following process steps:
a) nitration of benzene with nitric acid or a mixture of nitric acid and sulfuric acid and separating off of the aqueous phase;
b) washing of the organic process product obtained in step a);
c) alkaline washing of the washed organic process product obtained in step b);
d) optional separation of benzene and/or nitrobenzene out of the alkaline waste water obtained in step c);
e) optional stripping of the alkaline waste water obtained in step c) or step d).

## Revendications

1. Procédé de traitement d'une eau résiduaire alcaline, qui est formée lors du lavage de nitrobenzène brut, obtenu par nitration de benzène, selon lequel
(i) l'eau résiduaire alcaline est portée à une température de 150 °C à 500 °C avec exclusion de l'oxygène à une pression élevée par rapport à la pression atmosphérique ;
(ii) l'eau résiduaire obtenue en (i) est mélangée avec une base ;
(iii) l'eau résiduaire obtenue en (ii) est davantage purifiée par extraction avec un gaz d'extraction, puis le courant de gaz d'extraction chargé avec les impuretés est refroidi à une température de 10 °C à 60 °C.

2. Procédé selon la revendication 1, selon lequel le chauffage de l'eau résiduaire alcaline à l'étape (i) est réalisé à une pression absolue de 50 bar à 350 bar.

3. Procédé selon la revendication 1 ou 2, selon lequel le chauffage de l'eau résiduaire alcaline à l'étape (i) est réalisé pendant une durée de 5 minutes à 120 minutes.

4. Procédé selon la revendication 3, selon lequel, après le chauffage, l'eau résiduaire alcaline est refroidie à une température de 60 °C à 100 °C.

5. Procédé selon l'une quelconque des revendications 1 à 4, selon lequel la base utilisée à l'étape (ii) est une solution aqueuse choisie dans le groupe constitué par l'hydroxyde de lithium, l'hydroxyde de sodium, l'hydroxyde de potassium et l'hydroxyde de rubidium.

6. Procédé selon l'une quelconque des revendications 1 à 5, selon lequel la base est ajoutée à l'étape (ii) de manière à ajuster un pH d'au moins 12.

7. Procédé selon l'une quelconque des revendications 1 à 6, selon lequel l'extraction à l'étape (iii) est réalisée à une pression absolue de 0,5 bar à 2 bar et à une température de 80 °C à 120 °C.

8. Procédé selon l'une quelconque des revendications 1 à 7, selon lequel le gaz d'extraction est la vapeur d'eau.

9. Procédé selon l'une quelconque des revendications 1 à 8, selon lequel les constituants organiques sont éliminés du courant obtenu à l'étape (iii) après le refroidissement du courant de gaz d'extraction chargé avec des impuretés à une température de 10 °C à 60 °C, de manière à obtenir un courant appauvri en constituants organiques.

10. Procédé selon la revendication 9, selon lequel le courant appauvri en constituants organiques est recyclé d'en partie à en totalité dans l'extraction (iii).

11. Procédé selon la revendication 9 ou 10, selon lequel le courant appauvri en constituants organiques, s'il n'est pas recyclé dans l'extraction (iii), est introduit directement dans un traitement de l'eau résiduaire.

12. Procédé selon l'une quelconque des revendications 1 à 11, selon lequel l'eau résiduaire extraite obtenue à l'étape (iii) est introduite directement dans un traitement de l'eau résiduaire.

13. Procédé selon l'une quelconque des revendications 1 à 12, selon lequel l'eau résiduaire alcaline utilisée à l'étape (i) provient de l'étape c) ou de l'étape d) ou de l'étape e) des étapes de procédé suivantes :
a) la nitration de benzène avec de l'acide nitrique ou un mélange d'acide nitrique et d'acide sulfurique et la séparation de la phase aqueuse ;
b) le lavage du produit de procédé organique obtenu à l'étape a) ;
c) le lavage alcalin du produit de procédé organique lavé obtenu à l'étape b) ;
d) la séparation optionnelle de benzène et/ou de nitrobenzène de l'eau résiduaire alcaline obtenue à l'étape c) ;
e) l'extraction optionnelle de l'eau résiduaire alcaline obtenue à l'étape c) ou à l'étape d).
